# EUROPÄISCHE PATENTSCHRIFT

(11) **EP 1 684 847 B1**
(45) Veröffentlichungstag und Bekanntmachung des Hinweises auf die Patenterteilung: **22.06.2011**
(21) Anmeldenummer: 04790743.1
(22) Anmeldetag: 22.10.2004
(51) Int. Cl.: A61M 39/10, F16L 37/14

(54) **KONNEKTOR FÜR DIALYSEPORT**
CONNECTOR FOR A DIALYSIS PORT
CONNECTEUR DE PORT DE DIALYSE

(30) Priorität: 10.11.2003 DE 10352859
(43) Veröffentlichungstag der Anmeldung: 02.08.2006
(73) Patentinhaber: Fresenius Medical Care Deutschland GmbH, 61352 Bad Homburg (DE)
(72) Erfinder: SCHMIDT, Helmut, 66649 Oberthal (DE)
(74) Vertreter: Ziermann, Oliver
(86) Internationale Anmeldenummer: PCT/EP2004/011947
(87) Internationale Veröffentlichungsnummer: WO 2005/046785

(56) Entgegenhaltungen:
- EP-A- 0 406 587
- EP-A- 0 442 310
- US-A- 4 331 540
- US-A- 5 052 725
- US-A- 5 813 703
- US-A- 5 997 048

## Beschreibung

Die Erfindung betrifft einen Konnektor zur Verbindung eines Dialysatports eines Blutdialysators mit einer dialysatführenden Leitung nach dem Oberbegriff des Anspruchs 1.

Während einer Hämodialysebehandlung wird Blut eines Patienten mit Hilfe eines extrakorporalen Kreislaufs über einen Blutdialysator geleitet. Solche Dialysatoren bestehen heutzutage aus einem Bündel von Tausenden von semipermeablen Hohlfasermembranen, durch deren Inneres das Blut geleitet wird. An der Außenseite der Hohlfaser wird eine Reinigungsflüssigkeit - die Dialysierflüssigkeit oder das Dialysat - zirkuliert, in die die aus dem Blut zu entfernenden Stoffe per Diffusion und/oder Konvektion übertreten.

Ein solcher Blutdialysator weist im Allgemeinen vier als Port bezeichnete Flüssigkeitsanschlüsse auf: zwei für das Blut und zwei für das Dialysat. An diese Ports wird das Schlauchsystem des extrakorporalen Blutkreislaufs und das dialysatführende Leitungssystem im Sinne je einer zuführenden und einer abführenden Leitung angeschlossen. Zum Zwecke einer einheitlichen Verwendung werden für die Blutports einerseits und für die Dialysatports andererseits genormte Portformen verwendet. Während das Verbindungssystem für das Blutschlauchsystem auf Einmalartikel ausgelegt ist, werden bei vielen Hämodialysegeräten zur Behandlung der chronischen Niereninsuffizienz für die dialysatfiihrenden Leitungen Schläuche eingesetzt, die wiederverwendet werden. Als Verbindungssystem wird die sogenannte Hansen-Kupplung eingesetzt. Bei der Hansen-Kupplung wird über ein metallenes Kugellagerelement eine Arretierung der Verbindung zum Dialysatport hergestellt. Letzterer besteht nach DIN 58352 aus einem im Wesentlichen rohrförmigen Fortsatz, der vor dem Ende des Ports eine umlaufende Hinterschneidung im Sinne eines verringerten Außendurchmessers aufweist, in die die Kugeln des Kugellagerelementes einrasten. Die dialysatführenden Leitungen werden zwischen einzelnen Behandlungen zusammen mit dem restlichen Dialysatkreislauf gespült und gereinigt.

Bei anderen Hämodialysegeräten werden jedoch auch Wegwerfschlauchsets für die dialysatführenden Leitungen verwendet. In diesem Fall ist es zweckmäßig, für den entsprechenden Konnektor eine andere Gestaltung als die Hansen-Kupplung vorzunehmen, insbesondere wenn es sich bei dem Konnektor ebenfalls um ein Wegwerfteil handeln soll.

In der EP 0 442 310 A1 werden Dialysatorports eines Blutdialysators beschrieben, die sowohl die Ankoppelung von Hansen-Kupplungen als auch von anderen Konnektoren ermöglichen. Dafür ist der Dialysatorport mit einem Gewinde versehen, auf denen ein entsprechendes Konnektorgegenstück aufgeschraubt werden kann.

In der US 5,052,725 ist ein einstückiges weibliches Verbindungselement mit einer Vorderseite und einer Rückseite beschrieben. Das weibliche Verbindungselement definiert einen Pfad für den Fluss von Fluiden durch das Verbindungselement. Ein männliches Verbindungselement, das eine Vorder- und eine Rückseite und einen Pfad für ein Fluid durch das männliche Verbindungselement aufweist, ist in eine Vorderseite des weiblichen Verbindungselements einschiebbar. Ein einstückiges Befestigungselement ist verschiebbar in einem Schlitz des weiblichen Verbindungselements angeordnet und kann von einer Verbindungsposibon, in der das männliche Verbindungselement verbunden wird, und einer Abklemm- oder Trennposition in der das männliche Verbindungselement lösbar ist, verschoben werden.

Eine Schraubbewegung zur Konnektierung ist insofern nachteilig, als dass sich der Endpunkt der Schraubbewegung nur schwer erfassen lässt. Bei nur ungenügendem Aufschrauben des Konnektors kann es zu Leckagen kommen, während es andererseits durch einen zu großen Krafteintrag am Ende der Konnektierung schnell zur Beschädigung der Dichtungselemente kommen kann, die in diesen Konnektoren im Allgemeinen vorgesehen sind. Zusätzlich kann dieser Konnektor nur eingesetzt werden, wenn die Ports an den Dialysatoren ebenfalls entsprechend gestaltet sind, d.h. es sind auch gestalterische Maßnahmen am Gegenstück des Konnektors notwendig.

Der Erfindung liegt die Aufgabe zugrunde, einen gattungsgemäßen Konnektor dergestalt weiterzubilden, dass er als Einmalartikel geeignet herstellbar ist und gleichzeitig eine einfache und verlässliche Konnektierung einer dialysatführenden Leitung an einen Dialysatport eines Blutdialysators ermöglicht, ohne konstruktive Maßnahmen am Dialysator selbst zu erfordern.

Nach der Lehre der Erfindung wird diese Aufgabe durch einen Konnektors mit den Merkmalen des Anspruches 1 gelöst. Gestalterische Ausführungsformen der Erfindung sind Gegenstand der Unteransprüche.

Die Erfindung basiert auf der Beobachtung, dass der nach DIN 58352 gestaltete Dialysatorport eine umlaufende Hinterschneidung auf der Außenseite aufweist. Diese Hinterschneidung kann von einem am erfindungsgemäß gestalteten Konnektor vorgesehenen Schiebeelement zur Arretierung des Konnektors verwendet werden. Eine Umgestaltung des dialysatorseitigen Ports ist in diesem Fall nicht notwendig.

Weitere Einzelheiten und Vorteile der Erfindung werden anhand eines in den Zeichnungen dargestellten Ausführungsbeispiels näher beschrieben. Es zeigen:
Fig. 1 eine Seitenansicht einer Ausführungsform des erfindungsgemäßen Konnektors mit dem Schiebeelement in der ersten Position,
Fig. 2a die Ansicht des Schnitts AA in Fig. 1,
Fig. 2b die zu Fig. 2a entsprechende Ansicht mit dem Schiebeelement in der zweiten Position,
Fig. 3a einen Schnitt des Konnektors von Fig. 1 längs der Symmetrieachse und in Sicht der Schieberichtung des Schiebeelementes, wobei das Schiebeelement in der ersten Position ist und
Fig. 3b die zu Fig. 3a entsprechenden Ansicht mit dem Schiebeelement in der zweiten Position.

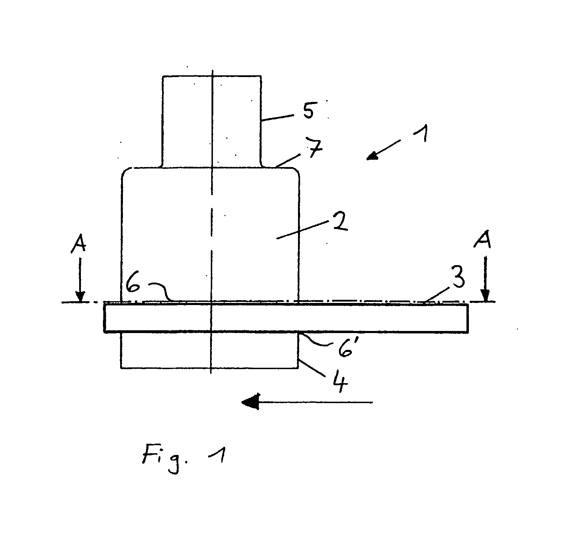

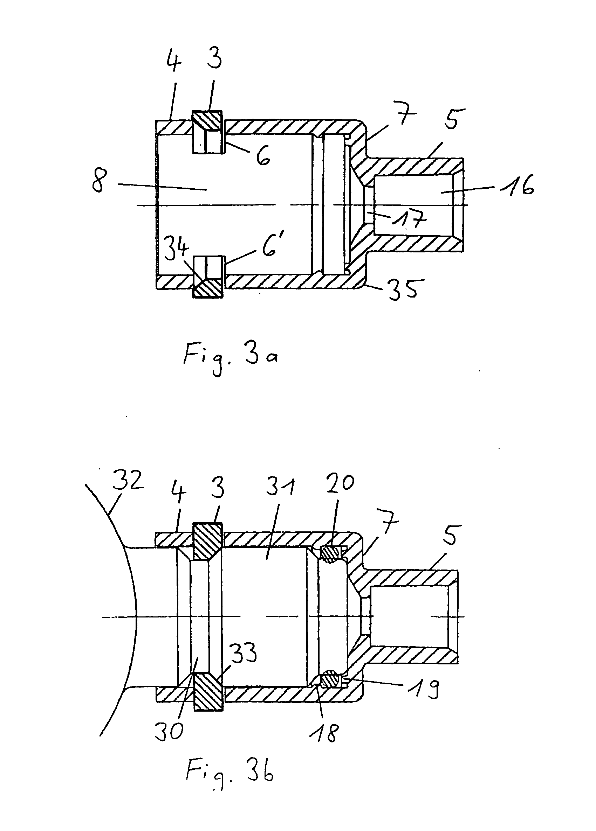

Fig. 1 zeigt eine Ausführungsform des erfindungsgemäßen Konnektors 1 zur Verbindung eines Dialysatports eines Blutdialysators mit einer dialysatführenden Leitung (nicht gezeigt) in der Seitenansicht. Der Konnektor 1 besteht aus einem Grundkörper 2 und einem Schiebeelement 3. Der Grundkörper 2 setzt sich aus einer ersten zylindrischen Hülse 4, die sich an dem ersten, an den Dialysatorport zu konnektierenden Ende des Konnektors 1 befindet, und einer zweiten zylindrischen Hülse 5 zusammen, die an dem mit der dialysatführenden Leitung zu konnektierenden, zweiten Ende des Konnektors 1 angeordnet ist. Der Außendurchmesser der ersten Hülse ist dabei größer als der Außendurchmesser der zweiten Hülse, was durch die Bemessungen der zu verbindenden Leitungen bedingt ist. Beide Hülsen gehen in einem Verbindungsbereich 7 fluiddicht ineinander über, wie dies anhand von Fig. 3a und 3b erkennbar ist. Durch den Grundkörper des Konnektors zieht sich ein in Fig. 1 nicht gezeigtes Lumen, durch das im Falle der Konnektion zum Teil das Dialysat fließt und dass zum anderen Teil den Dialysatport des Dialysators aufnimmt.

In dem ersten Ende 4 des Konnektors 1 ist eine Aussparung 6 an der der Ansicht von Fig. 1 zugewandten Seite sowie eine Aussparung 6' an der nicht gezeigten gegenüberliegenden Seite zur Aufnahme des Schiebeelementes 3 vorgesehen. Das Schiebeelement 3 befindet sich in Fig. 1 in der ersten Position. Es ist in Pfeilrichtung in die zweite Position bewegbar.

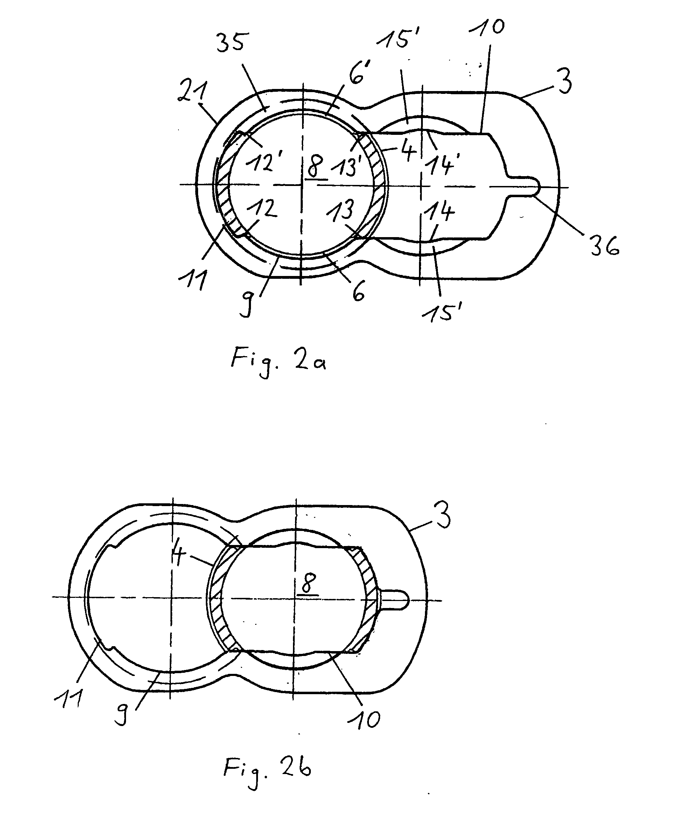

Fig. 2a zeigt die Ansicht von Fig. 1 in der Schnittansicht A-A. Das Schiebeelement 3 befindet sich entsprechend in der ersten Position. Dabei ist das Lumen 8 der ersten zylindrischen Hülse 4 sowie die Aussparungen 6 und 6' erkennbar, in denen das Schiebeelement 3 von der ersten in die zweite Position bewegt werden kann.

Das Schiebeelement 3 weist ferner eine schlüssellochartige Gesamtöffnung auf, die sich aus einer ersten, runden Öffnung 9 und einer sich in Schieberichtung anschließenden zweiten, länglichen Öffnung 10 zusammensetzt. In der in Fig. 2a gezeigten ersten Position des Schiebeelementes 3 ist die erste Öffnung 9 konzentrisch mit dem Lumen 8 des ersten Endes 4 des Konnektors 1 angeordnet, so dass das Lumen 8 nicht eingeengt wird. Räumlich gegenüber der zweiten Öffnung 10 ist die erste Öffnung 9 in einem Bereich 11 ein wenig erweitert, so dass sich an der Begrenzung der erweiterten Öffnung 11 Vorsprünge 12 und 12' ergeben, die nach Innen gerichtet sind. In den Aussparungen 6 und 6' sind zu diesen Vorspüngen komplementäre Aussparungen vorsehen, in die die Vorspünge 12 und 12' einrasten können. Diese Aussparungen sind zusätzlich symmetrisch an der gegenüberliegenden Seite der ersten Hülse 4 vorgesehen und dort mit 13 und 13' beziffert. Dies hat den Vorteil, dass der Grundkörper 2 bezogen auf die zur Schieberichtung senkrechte Ebene spiegelsysmmetrisch ist, was ein aufwendiges Orientieren des Grundkörpers 2 bei der Montage mit dem Schiebeelement 3 überflüssig macht.

Durch Einrasten der Vorsprünge 12 und 12' kann der Konnektor 1 mit dem Schiebeelement 3 in der ersten Position ausgeliefert werden, ohne dass der Benutzer sich durch Betätigung des Schiebeelementes 3 von der Ausrichtung der ersten Öffnung 9 mit dem Lumen 8 bei Gebrauch des Konnektors überzeugen muss. Der Bügel 21 des Schiebeelementes 3 kommt dabei außerhalb des Grundkörpers 2 zum Anschlag an diesen.

In der zweiten Öffnung 10 ist ein erweiterter Öffnungsbereich 14 und 14' vorgesehen, der konzentrisch mit dem Lumen 8 verläuft, wenn das Schiebeelement in der zweiten Position ist (Fig. 2b). In dieser Stellung verengt die zweite Öffnung 10 das Lumen 8 in Richtung der Aussparungen 6 und 6'. Die Konturen der Öffnungsbereiche 14 und 14' sind denen des Dialysatports des Blutdialysators nachempfunden. Durch die geringfügig eingeschränkte Weite der Öffnung 10 unmittelbar neben den Bereichen 14 und 14' wird das Schiebeelement 3 auch in der zweiten Position - bei eingesetztem Dialysatport (in Fig. 2b nicht gezeigt) - eingerastet festgelegt.

Die zu überwindende Federkraft kann dabei durch die geeignete Dimensionierung einer Verjüngung 36 eingestellt werden. Da zudem sowohl der Grundkörper 2 als auch das Schiebeelement 3 vorzugsweise aus Kunststoff hergestellt werden, lassen sich die Vorspünge 12 und 12' sowie die Öffnungsbereiche 14 und 14' leicht so dimensionieren und ein Material so wählen, dass die Rastvorgänge ohne übermäßige Kraftanwendung und doch mit verlässlicher und bemerkbarer, insbesondere hörbarer, Arretierung durchführbar sind.

An die Öffnungsbereiche 14 und 14' schließen sich Bereiche15 und 15' an, an denen sich die Wandstärke des Schiebeelementes 3 im Sinne einer Schräge zur Öffnung 10 hin verringert. Die Funktion dieser Schräge wird später anhand von Fig. 3b erläutert werden.

In Fig. 3a ist ein Schnitt quer zur Symmetrieachse des Konnektors 1 in Richtung der Schieberichtung des Schiebeelementes 3 gezeigt, wobei sich das Schiebeelement 3 in der ersten Position befindet. In dieser Ansicht ist auch die erste Hülse 4 sowie die zweite Hülse 5 des Grundkörpers 2 erkennbar. Die erste Hülse 4 wird durch ein Lumen 8 durchzogen, die zweite Hülse 5 durch ein Lumen 16 mit kleinerem Durchmesser. Zwischen beiden Lumen liegt eine Verengung 17. Im Verbindungsbereich zwischen der ersten und der zweiten Hülse schließt der einstückige Grundkörper durch eine ringartige Wandung 7 ab. Am ersten Ende 4 des Grundkörpers 2 sind die Aussparungen 6 und 6' zur Aufnahme des Schiebeelementes 3 erkennbar. Da sich das Schiebeelement 3 in der ersten Position befindet, wird das Lumen 8 in seiner Öffnung durch das Schiebeelement nicht beeinträchtigt.

Das Schiebeelement 3 ist dabei ausreichend flexibel hergestellt, um sich in dieser Stellung leicht auf den Grundkörper 2 durch Spreizung der schenkelartigen Begrenzungen der ersten Öffnung 9 montieren zu lassen. Dies wird durch eine umlaufende Schräge 34 erleichtet, die gestrichelt auch in Fig. 2a und 2b angedeutet ist. Bei der Montage des Schiebeelementes 3 auf den Grundkörper 2 von der Seite der zweiten Hülse 5 her wird das Schiebeelement 3 mit der runden Öffnung 9 auf den Grundköper 2 gedrückt, so dass die angeschrägte Fläche 34 auf die abgerundeten Begrenzungen 35 des Grundkörpers 2 in dem Verbindungsbereich 7 treffen. Dadurch wird das Vorschieben des Schiebeelementes 3 bis zur Einrastung in die Aussparungen 6 und 6' erleichtert. Alternativ kann die Schräge 34 auch auf der anderen Seite des Schiebeelementes 3 vorgesehen sein, wenn die Montage von seiten der ersten Hülse 4 erfolgen soll.

Mit dem Schiebeelement 3 in der ersten Position kann der erfindungsgemäße Konnektor auf den Dialysatport aufgeschoben werden. Der Konnektor 1 wird dann durch das Verschieben des Schiebelementes 3 in die zweite Position arretiert (Fig. 3b). Das Schiebeelement 3 hinterfasst dabei die in dem Dialysatport 31 vorgesehene, als umlaufende Nut ausgeführte Hinterschneidung 30. Der Dialysatports 31 ist Teil eines Dialysatorgehäuses 32.

Die Nut 30 ist mit einer dem Ende des Ports zugeneigten Schräge 33 versehen. Zu dieser Schräge 33 sind die Bereiche 15 und 15' formschlüssig ausgeführt. Dadurch wird ein axialer Krafteintrag erreicht, der zur Fixierung des Konnektors 1 an den Port 31 beiträgt. Der Dialysatport 31 wird dabei gegen die Stirnseite 7 des Grundkörpers 2 gedrückt, die gleichzeitig als Anschlag für den Port dient. Zusätzlich können umlaufende Vorspünge 18 und 19 an der Innenseite des Lumens 8 vorgesehen sein, die zur Fixierung eines Dichtungselementes 20 dienen, das als O-Ring ausgeführt sein kann. Dieses Dichtungselement 20 umschließt das äußere Ende des Ports 31, dass an dieser Stelle einen entsprechend verringerten Außendurchmesser aufweist, um eine zuverlässige Abdichtung des Konnektors 1 gegenüber dem Port 31 zu erreichen.

Die dialysatführende Leitung - z.B. in Form eines Schlauches - kann im einfachsten Fall auf die zweite Hülse 5 aufgesteckt werden. Je nach Anforderung kann dabei eine Überwurfklemmung vorgesehen sein. Es können auch andere Verbindungsmethoden oder nicht lösbare Verbindungen, die vormontiert sind, z.B. durch Klebung oder Verschweißung, verwendet werden. Hierfür sind dem Fachmann unterschiedlichste handwerkliche Ausgestaltungen geläufig.

Der erfindungsgemäße Konnektor ermöglicht eine Konnektierung einer dialysatführenden Leitung an einen handelsüblichen Port eines Blutdialysators, die in der Handhabung einfach und verlässlich ist. Er erfordert zudem nur wenige Einzelteile und kann kostengünstig aus Kunststoffen in Spritzgusstechnik hergestellt werden. Der Konnektor kann auch bei anderen Ports eingesetzt werden, deren Stutzen in das erste Ende des Konnektors einführbar sind und die eine Hinterschneidung aufweisen, die mit Hilfe des Schiebeelementes zur Arretierung hinterfasst werden können. Dies gilt insbesondere für alle Ports nach DIN 58352.

## Patentansprüche

1. Konnektor (1) zur Verbindung eines Dialysatports (31) eines Blutdialysators (32) mit einer dialysatfuhrenden Leitung mit
einem den Konnektor (1) durchlaufenden Lumen (8, 16),
einem das Lumen (8) umschließenden ersten Ende (4) des Konnektors (1), das geeignet ist, in das Lumen (8) den Dialysatport (31) aufzunehmen,
einem das Lumen (16) umschließenden zweiten Ende (5) des Konnektors (1), das geeignet ist, mit der dialysatführenden Leitung verbunden zu werden,
**dadurch gekennzeichnet,**
**dass** am ersten Ende (4) zwei Aussparungen (6, 6') mit einem von den Aussparungen aufgenommene Schiebeelement (3) vorgesehen sind,
wobei das Schiebeelement (3) zwischen einer ersten und einer zweiten Position senkrecht zur Richtung des Lumens (8) im ersten Ende (4) verschiebbar ist,
wobei das Schiebeelement (3) in der ersten Position das Lumen (8) des ersten Endes (4) nicht durchringt und in der zweiten Position das Lumen (8) des ersten Endes (4) einengt, so dass der Konnektor (1) mit dem Schiebeelement (3) in der ersten Position auf den Dialysatport (31) aufgesteckt werden und in der zweiten Position durch eine Hinterschneidung (30) am Dialysatport (31) an diesem arretiert werden kann,
wobei das Schiebeelement (3) eine erste Öffnung (9) aufweist, die das Lumen (8) im ersten Ende (4) in der ersten Position nicht einengt, und eine sich an die erste Öffnung in Schieberichtung anschließende zweite Öffnung (10), die das Lumen (8) im ersten Ende (4) in Richtung der Aussparungen (6,6') in der zweiten Position einengt,
wobei die erste Öffnung (9) eine runde Fort und die zweite Öffnung (10) eine längliche Form ausweisen, so dass die Gesamtöffnung schlüssellochförmig ist,
und wobei die zweite Öffnung (10) senkrecht zur Schieberichtung mit einem den Dialysatorport (31) aufnehmenden erweiterten Öffnungsbereich (14, 14') zur Einrastung des Schiebeelementes (3) in der zweiten Position versehen ist.

2. Konnektor nach Anspruch 1, **dadurch gekennzeichnet, dass** der Konnektor (1) neben dem Schiebeelement (3) aus einem Grundkörper (2) besteht, der sich aus zwei miteinander verbundenen, im wesentlichen zylindrischen Hülsen (4, 5) zusammensetzt, wobei die erste Hülse das erste Ende und die zweite Hülse das zweite Ende ist.

3. Konnektor nach Anspruch 2, **dadurch gekennzeichnet, dass** der Außendurchmesser der ersten Hülse (4) größer als der Außendurchmesser der zweiten Hülse (5) ist.

4. Konnektor nach Anspruch 2 oder 3, **dadurch gekennzeichnet, dass** die erste Hülse (4) geignet ist, einen Port nach DIN 58352 aufzunehmen.

5. Konnektor nach einem der Ansprüche 2 bis 4, **dadurch gekennzeichnet, dass** das Lumen (8) in der ersten Hülse (4) einen größeren Durchmesser als das Lumen (16) in der zweiten Hülse (5) hat.

6. Konnektor nach einem der Ansprüche 2 bis 5, **dadurch** gekennzeichnt, dass der Konnektor (1) im Verbindungsbereich der zwei Hülsen (4, 5) mit einem Anschlag (7) für den Dialysatorport (31) versehen ist.

7. Konnektor nach Anspruch 6, **dadurch gekennzeichnet, dass** an der Innenwand der ersten Hülse (4) in der Nähe des Anschlags (7) ein Dichtungselement (20) zur Abdichtung des Konnektors (1) gegenüber dem Dialysatorport (31) vorgesehen ist.

8. Konnektor nach einem der vorhergehenden Ansprüche, **dadurch gekennzeichnet, dass** sich zwischen dem ersten (4) und dem zweiten Ende (5) eine Verengungstelle (17) im Lumen (8, 16) vorgesehen ist.

9. Konnektor nach Anspruch 1, **dadurch gekennzeichnet, dass** die erste Öffnung (10) Einrastvorsprunge (12, 12') aufweist, die zum Zwecke des Einrastens des Schiebeelementes (3) in der ersten Position mit komplementären Ausnehmungen (13, 13') an dem ersten Ende (4) des Konnektors (1) einrasten können.

10. Konnektor nach Anspruch 9, **dadurch gekennzeichnet, dass** die komplementären Ausnehmungen (13, 13') an dem ersten Ende (4) des Konnektors (1) an der in Schieberichtung gegenüberliegenden Seite des Lumens (8) symmetrisch zusätzlich vorgesehen sind.

11. Konnektor nach Anspruch 1, **dadurch gekennzeichnet, dass** die längliche Begrenzung der zweiten Öffnung (10) zur Öffnung hin eine sich mit einer Schräge verjüngende Wandstärke aufweist, die mit einer entsprechenden Schräge (33) an der Hinterschneidung (30) des Dialysatorports (31) formschlüssig ist.

## Claims

1. A connector (1) for connecting a dialysate port (31) of a blood dialyzer (32) to a dialysate-carrying line, comprising
a lumen (8, 16) which passes through the connector (1),
a first end (4) of the connector (1) surrounding the lumen (8), suitable for accommodating the dialysate port (31) in the lumen (8),
a second end (5) of the connector (1) surrounding the lumen (16), suitable for being connected to the dialysate-carrying line,
**characterized in that**
two recesses (6, 6') on the first end (4) are provided with a sliding element (3), which is accommodated in the recesses,
wherein the sliding element (3) is displaceable between a first position and a second position perpendicular to the direction of the lumen (8) in the first end (4),
wherein the sliding element (3) in the first position does not penetrate through the lumen (8) of the first end (4), and in the second position the lumen (8) of the first end (4) is constricted, so that the connector (1) with the sliding element (3) can be attached to the dialysate port (31) in the first position and can be locked on the dialysate port (31) by an undercut (30) on same in the second position,
wherein the sliding element (3) has a first opening (9), which in the first position does not constrict the lumen (8) in the first end (4) and a second opening (10), which in the second position is connected to the first opening in the direction of sliding and constricts the lumen (8) in the first end (4) in the direction of the recesses (6, 6'),
wherein the first opening (9) has a round shape and the second opening (10) has an elongated shape, so that the entire opening has a keyhole shape,
and wherein the second opening (10) is perpendicular to the direction of sliding, having a widened opening area (14, 14') accommodating the dialyzer port (31) for engagement of the sliding element (3) in the second position.

2. The connector according to Claim 1, **characterized in that** the connector (1), in addition to the sliding element (3), consists of a base body (2), which is composed of two essentially cylindrical sleeves (4, 5) that are connected to one another, wherein the first sleeve is the first end and the second sleeve is the second end.

3. The connector according to Claim 2, **characterized in that** the outside diameter of the first sleeve (4) is larger than the outside diameter of the second sleeve (5).

4. The connector according to Claim 2 or 3, **characterized in that** the first sleeve (4) is suitable for accommodating a port according to DIN 58352.

5. The connector according to any one of Claims 2 to 4, **characterized in that** the lumen (8) in the first sleeve (4) has a larger diameter than the lumen (16) in the second sleeve (5).

6. The connector according to any one of Claims 2 to 5, **characterized in that** the connector (1) is provided with a stop (7) for the dialyzer port (31) in the connecting area of the two sleeves (4, 5).

7. The connector according to Claim 6, **characterized in that** a sealing element (20) for sealing the connector (1) with respect to the dialyzer port (31) is provided on the inside wall of the first sleeve (4) in the vicinity of the stop (7).

8. The connector according to any one of the preceding claims, **characterized in that** a constriction site (17) is provided in the lumen (8, 16) between the first end (4) and the second end (5).

9. The connector according to Claim 1, **characterized in that** the first opening (10) has locking protrusions (12, 12') which can engage with complementary recesses (13, 13') on the first end (4) of the connector (1) for the purpose of locking the sliding element (3) in the first position.

10. The connector according to Claim 9, **characterized in that** the complementary recesses (13, 13') on the first end (4) of the connector (1) are additionally provided symmetrically on the side of the lumen (8) opposite the direction of sliding.

11. The connector according to Claim 1, **characterized in that** the elongated border of the second opening (10) with respect to the opening has a wall thickness which tapers with a slope and is in form-fitting engagement with a corresponding slope (33) on the undercut (30) of the dialyzer port (31).

## Revendications

1. Connecteur (1) pour le raccordement d'un port à dialysat (31) d'un dialyseur sanguin (32) à une canalisation de dialysat comportant un lumen (8, 16) traversant le connecteur (1),
une première extrémité (4) enclavant le lumen (8) du connecteur (1), qui est apte à recevoir le port à dialysat (31) dans le lumen (8),
une seconde extrémité (5) enclavant le lumen (16) du connecteur (1), qui est apte à être raccordée à la canalisation de dialysat,
**caractérisé en ce que**,
il est prévu à la première extrémité (4) deux échancrures (6, 6') avec un élément coulissant (3) reçu par les échancrures,
l'élément coulissant (3) étant déplaçable entre une première et une seconde position perpendiculairement au sens du lumen (8) dans la première extrémité (4),
l'élément coulissant (3), dans la première position, ne traversant pas le lumen (8) de la première extrémité (4) et, dans la seconde position, rétrécissant le lumen (8) de la première extrémité (4), de sorte que le connecteur (1) peut, dans la première position, être posé avec l'élément coulissant (3) sur le port à dialysat (31) et peut, dans la seconde position, être arrêté par une contre-dépouille (30) du port à dialysat (31) au niveau de ce dernier,
l'élément coulissant (3) présentant une première ouverture (9) qui ne rétrécit pas le lumen (8) dans la première extrémité (4) dans la première position et une deuxième ouverture (10) se raccordant à la première ouverture dans le sens de coulissement et qui rétrécit le lumen (8) dans la première extrémité (4) dans le sens des échancrures (6, 6') dans la seconde position,
la première ouverture (9) présentant une forme ronde et la seconde ouverture (10) une forme allongée, de sorte que l'ensemble de l'ouverture a la forme d'un trou de serrure,
et la seconde ouverture (10) étant pourvue, perpendiculairement au sens de coulissement, d'une zone d'ouverture (14, 14') élargie recevant le port à dialysat (31) pour l'enclenchement de l'élément coulissant (3) dans la seconde position.

2. Connecteur selon la revendication 1, **caractérisé en ce que** le connecteur (1) est composé, outre de l'élément coulissant (3), d'un corps de base (2) qui est constitué de deux manchons sensiblement cylindriques (4, 5) reliés entre eux, le premier manchon étant la première extrémité et le second manchon la seconde extrémité.

3. Connecteur selon la revendication 2, **caractérisé en ce que** le diamètre extérieur du premier manchon (4) est supérieur au diamètre extérieur du second manchon (5).

4. Connecteur selon la revendication 2 ou 3, **caractérisé en ce que** le premier manchon (4) est apte à recevoir un port conforme à la DIN 58352.

5. Connecteur selon une des revendications 2 à 4, **caractérisé en ce que** le lumen (8) du premier manchon (4) a un diamètre supérieur au lumen (16) du second manchon (5).

6. Connecteur selon une des revendications 2 à 5, **caractérisé en ce que** le connecteur (1), dans la zone de raccordement des deux manchons (4, 5), est pourvu d'une butée (7) pour le port à dialysat (31).

7. Connecteur selon la revendication 6, **caractérisé en ce que**, sur la paroi intérieure du premier manchon (4), à proximité de la butée (7), il est prévu un élément d'étanchéité (20) pour isoler le connecteur (1) du port à dialysat (31).

8. Connecteur selon une des revendications précédentes, **caractérisé en ce qu'**un point de rétrécissement (17) est prévu dans le lumen (8, 16) entre la première (4) et la seconde extrémité (5) .

9. Connecteur selon la revendication 1, **caractérisé en ce que** la première ouverture (10) présente des saillies d'enclenchement (12, 12') qui, dans le but d'enclencher l'élément coulissant (3) dans la première position, peuvent s'enclencher dans des échancrures complémentaires (13, 13') à la première extrémité (4) du connecteur (1).

10. Connecteur selon la revendication 9, **caractérisé en ce que** les échancrures complémentaires (13, 13') sont de plus prévues symétriques au niveau de la première extrémité (4) du connecteur (1) sur la face opposée dans le sens de coulissement du lumen (8) .

11. Connecteur selon la revendication 1, **caractérisé en ce que** la limite longitudinale de la seconde ouverture (10) présente une épaisseur de paroi se rétrécissant en biais qui est en correspondance géométrique avec un biais correspondant (33) situé au niveau de la contre-dépouille (30) du port à dialysat (31).
